# EUROPÄISCHE PATENTSCHRIFT

(11) **EP 3 493 858 B1**
(45) Veröffentlichungstag und Bekanntmachung des Hinweises auf die Patenterteilung: **07.06.2023**
(21) Anmeldenummer: 17746103.5
(22) Anmeldetag: 01.08.2017
(51) Int. Cl.: A61L 27/04, A61L 27/24, A61L 27/36, A61L 27/58

(54) **IMPLANTAT, VORZUGSWEISE ZUM BEHANDELN EINES AZETABULUMDEFEKTS**
IMPLANT, PREFERABLY FOR THE TREATMENT OF AN ACETABULAR DEFECT
IMPLANT, DE PRÉFÉRENCE POUR LE TRAITEMENT D'UN DÉFAUT DE L'ACETABULUM

(30) Priorität: 02.08.2016 DE 102016214258
(43) Veröffentlichungstag der Anmeldung: 12.06.2019
(73) Patentinhaber: Aesculap AG, 78532 Tuttlingen (DE)
(72) Erfinder: KÖNIG, Silke, 78628 Rottweil (DE)
(74) Vertreter: Patentanwälte Ruff, Wilhelm, Beier, Dauster & Partner mbB
(86) Internationale Anmeldenummer: PCT/EP2017/069376
(87) Internationale Veröffentlichungsnummer: WO 2018/024700

(56) Entgegenhaltungen:
- EP-A1- 2 769 743
- WO-A1-2010/093333
- WO-A1-2013/077896
- WO-A1-2015/162559
- DE-A1-102005 018 644

## Beschreibung

Die Erfindung betrifft ein Implantat zum Behandeln eines Azetabulumdefekts sowie ein chirurgisches Kit, welches ein derartiges Implantat aufweist.

Die zunehmende Anzahl an Revisions- und Wechseloperationen nach Implantation von Hüfttotalendoprothesen stellt eine der größten Herausforderungen der modernen Versorgungschirurgie dar. Als insbesondere problematisch erweist sich dabei der mit Revisionen üblicherweise einhergehende Verlust an Knochengewebe im Azetabulum und am Femur.

Der Wiederherstellung von anatomisch knöchernen Verhältnissen und insbesondere biomechanisch korrekten Implantatlagen kommt daher eine besondere Bedeutung zu.

Grundsätzlich stehen unterschiedliche Behandlungsoptionen zur Verfügung.

Knochendefekte am Azetabulum können beispielsweise durch große zementfreie Pfannen, Revisionsringe, Abstützschalen oder Keramiktransplantate rekonstruiert oder alternativ durch modulare poröse Metallaugmentationen überbrückt werden.

Größere Knochendefekte des Azetabulums müssen vor Implantation einer Endoprothese sowohl im Rahmen einer Primärversorgung als auch im Rahmen einer Revision zunächst aufgefüllt werden. Bei Knochendefekten der Klasse Paprosky 2C (isolierte mediale Pfannenmigration, intakter Pfannenrand) und 3A (ausgeprägte superolaterale Pfannenmigration > 3 cm; 40-60 % Kontakt zum Implantatbett; unzureichende Stabilität; Risiko einer Beckendiskontinuität) erfolgt die Auffüllung in der Regel mittels Knochenchips.

Im Falle einer Revisionsoperation steht autologer Hüftknochen in der Regel nicht zur Verfügung, weswegen auf allogene Knochentransplantate zurückgegriffen werden muss.

Hierzu werden entweder aus Femurköpfen während der Operation hergestellte Knochenchips oder strukturelle, allogene Transplantate, wie beispielsweise Femurkopf-Augmentate, verwendet.

Ziel ist es, ein Knochenlager herzustellen, welches eine mechanisch sichere Implantation einer Gelenkspfanne erlaubt. Vor allem bei fortgeschrittenen, kombinierten Strukturdefekten mit einem Knochenvolumenverlust ist der Wiederaufbau durch Knochentransplantation unverzichtbar. Hierbei handelt es sich um einen technisch komplizierten Eingriff, welcher einen entsprechend erfahrenen und auch routinierten Operateur voraussetzt. Anderenfalls ist ein Versagen der implantierten Endoprothese vorprogrammiert.

Im Falle von Knochenchips werden diese schichtweise in die Defekthöhle eingebracht und impaktiert. Das Impaktieren der Knochenchips erfolgt häufig mittels eines sogenannten Nachschlägers (chirurgisches Instrument zum Impaktieren von Knochenchips oder Knochenspäne). Dabei soll eine gute Verzahnung der Knochenchips untereinander aber auch mit dem verbliebenen Knochengewebe erreicht werden. Besonders geeignet sind als sogenannte "Fresh-Frozen-Grafts" bezeichnete Knochenchips

Zur Herstellung derartiger Knochenchips wird zunächst ein bei -80 °C gelagerter Hüftkopf mit einer Säge in Scheiben zerlegt. Anschließend wird die Spongiosa ausgiebig gewaschen, um diese von Fett- und Blutresten zu befreien. Auch der glatte Knorpel wird restlos entfernt, da dieser das Durchwachsen von Knochenbälkchen verhindern kann.

Die Knochenchips werden anschließend - möglichst mit einer Luer-Knochenzange (gerade, übersetzungsfreie Zange mit Maulteilen in Hohlmeißelform zur Entfernung von Knochenkanten und Knorpel) oder in vergleichbaren Größen - präpariert.

Nahezu alle Knochenmühlen produzieren jedoch "Knochenmehl", welches mechanisch nicht ausreichend stabil ist. Die ideale Größe von Knochenchips beträgt 0.7 cm bis 1.0 cm. Bei der Herstellung mittels Knochenmühlen sind jedoch etwa nur Chipgrößen von 2 mm bis 6 mm erzielbar. Die höchste Stabilität wird durch spongiose Knochenchips in unterschiedlichsten Größen innerhalb des Größenbereichs von 0.7 cm bis 1.0 cm erreicht.

Vor der Einbringung muss das noch vorhandene natürliche Knochengegenlager vorsichtig angefräst oder angebohrt werden, um eine vitale Auflage für die Knochenchips zu erhalten. Nach der Einbringung sind die Knochenchips maximal zu verdichten ("impaction grafting"). Hierzu ist das Vorhandensein einer entsprechenden Begrenzung durch noch vorhandene knöcherne Defektwände Voraussetzung.

Die Inkorporation der Knochenchips erfolgt durch endochondrale Ossifikation (Verknöcherung von innen her) entlang der osteokonduktiven Grenze (Leitgerüst für natürliches Knochenwachstum) der Chips, wobei es im Laufe der Zeit zu einer Zunahme der mechanischen Stabilität kommt.

Nach Augmentation des natürlichen Knochens mit Knochenchips können sowohl zementierte als auch zementfreie Pfannen zur Anwendung kommen.

Um die Knochenchips bei größeren Defekten an Ort und Stelle zu halten und ein Gegenlager für das Impaktieren zu bekommen, werden oft sogenannte Revisionsnetze in den Knochendefekt eingebracht. Dadurch wird das Risiko beseitigt, dass die kompaktierten Knochenchips durch den Defekt hindurchgepresst werden und mithin Knochengewebe verletzen.

Die Revisionsnetze sind anatomisch derart geformt, dass sie für die Revisionsoperation direkt eingesetzt oder, falls erforderlich, auch durch Biegen angepasst werden können. Zusätzlich können die Netze mit vorkonfigurierten Löchern versehen sein, welche eine Fixierung mit Schrauben ermöglichen.

Die Revisionsnetze bestehen aus nicht abbaubarem Metall. Es besteht daher das Risiko, dass die Metallnetze fest mit den Knochenchips und dem Knochen verwachsen. Ist eine Revision sowie aus chirurgischen Gründen die Entfernung des Metallnetzes erforderlich, wird stets auch frischer Knochen mitentfernt, was eine Defektvergrößerung bedeutet und darüber hinaus den Knochenneuaufbau beeinträchtigen kann. Abgesehen davon können sich im Knochendefekt verbleibende Metallreste störend auf den operativen Eingriff sowie negativ auf den Behandlungserfolg auswirken.

Aus der DE 10 2005 018 644 A1 ist ein Implantat zur Behandlung von Röhrenknochendefekten bekannt, das ein textiles Flächengebilde mit einer mittigen konzentrischen kreisförmigen Öffnung zum Befestigen an einem Fixateur, wie beispielsweise einem Marknagel, sowie vier radial angeordnete, gleich verteilte Schlitze aufweist.

Der vorliegenden Erfindung liegt daher die Aufgabe zugrunde, ein alternatives Implantat zu den aus dem Stand der Technik bekannten Revisionsnetzen bereitzustellen, welches insbesondere die eingangs erwähnten Nachteile möglichst vermeidet.

Diese Aufgabe wird gelöst durch ein Implantat gemäß unabhängigem Anspruch 1 sowie ein chirurgisches Kit gemäß Anspruch 15. Bevorzugte Ausführungsformen sind in den abhängigen Ansprüchen definiert. Der Wortlaut sämtlicher Ansprüche wird hiermit durch ausdrückliche Bezugnahme zum Inhalt der vorliegenden Beschreibung gemacht.

Gemäß einem ersten Aspekt betrifft die Erfindung ein Implantat zum Behandeln und/oder Rekonstruieren, insbesondere Auskleiden und/oder Abdichten und/oder Unterfüttern und/oder wenigstens teilweisen Auffüllen, eines Azetabulumdefekts.

Das Implantat zeichnet sich besonders dadurch aus, dass es wenigstens ein Flächengebilde aufweist oder aus wenigstens einem Flächengebilde besteht, welches ein wenigstens teilweise in vivo abbaubares (degradierbares) oder ein wenigstens teilweise in vivo resorbierbares Material aufweist.

Durch das wenigstens teilweise in vivo abbaubare bzw. resorbierbare Material kann im Falle von erforderlichen Revisions- oder Wechseloperationen ein durch Knochenverwachsungen bedingter Knochenverlust gegenüber den gattungsgemäßen Revisionsnetzen signifikant reduziert oder gar vollständig vermieden werden.

Unter dem Ausdruck "Flächengebilde" soll im Sinne der vorliegenden Erfindung vorzugsweise ein Gebilde verstanden werden, welches in zwei seiner Dimensionen, bevorzugt seiner Länge und Breite, eine deutlich größere Abmessung besitzt als in seiner dritten Dimension, bevorzugt seiner Höhe oder Dicke.

Unter dem Ausdruck "wenigsten ein Flächengebilde" soll im Sinne der vorliegenden Erfindung ein Flächengebilde oder eine Mehrzahl von Flächengebilden, d.h. zwei oder mehr, beispielsweise zwei, drei oder vier, insbesondere zwei, Flächengebilde verstanden werden.

Unter dem Ausdruck "in vivo abbaubar" bzw. "in vivo degradierbar" soll im Sinne der vorliegenden Erfindung ein Material verstanden werden, welches in einem menschlichen oder tierischen Körper, insbesondere unter der Einwirkung von Enzymen, metabolisiert wird. Der Abbau des Materials kann dabei vollständig bis zur Mineralisierung, d.h. Freisetzung von chemischen Elementen und deren Einbau in anorganische Verbindungen, wie beispielsweise Kohlendioxid, Sauerstoff und/oder Ammoniak, verlaufen oder auf der Stufe von abbaustabilen Zwischen- oder Transformationsprodukten stehen bleiben.

Unter dem Ausdruck "in vivo resorbierbar" soll im Sinne der vorliegenden Erfindung ein Material verstanden werden, welches in einem menschlichen oder tierischen Körper durch lebende Zellen oder lebendes Gewebe, wie beispielsweise Nieren, aufgenommen wird, ohne dass ein Abbau oder ein nennenswerter Abbau des Materials stattfindet.

Unter dem Ausdruck "tierischer Körper" soll im Sinne der vorliegenden Erfindung der Körper eines nicht humanen Säugetiers, wie beispielsweise von Pferd, Kuh, Ziege, Schaf, Schwein oder einem Nagetier, wie beispielsweise Hase, Ratte oder Maus, verstanden werden.

Der Ausdruck "Material" kann im Sinne der vorliegenden Erfindung ein Material im Sinne eines Reinstoffs oder aber eine Materialmischung, d.h. eine Mischung aus verschiedenen Materialien, bedeuten. Bezüglich geeigneter Materialien sei auf die im Folgenden gemachten Ausführungen verwiesen.

In einer bevorzugten Ausführungsform handelt es sich bei dem Material um ein nur teilweise in vivo abbaubares oder nur teilweise in vivo resorbierbares Material. Ein nur teilweise in vivo abbaubares bzw. nur teilweise in vivo resorbierbares Material hat den Vorteil, dass hierdurch die biologische Rekonstruktion eines Azetabulumdefekts erleichtert wird.

In einer alternativen Ausführungsform handelt es sich bei dem Material um ein vollständig in vivo abbaubares oder vollständig in vivo resorbierbares Material. Ein vollständig in vivo abbaubares bzw. vollständig in vivo resorbierbares Material hat den Vorteil, dass es vollständig durch neugebildetes Knochengewebe ersetzt werden kann und nach Abbau bzw. Resorption des Materials kein Fremdmaterial in der ursprünglichen Defektzone verbleibt.

In einer weiteren Ausführungsform weist das Material eine randomisierte Faserstruktur, d.h. eine Struktur aus zufällig angeordneten und/oder orientierten Fasern, auf.

Insbesondere kann das Material eine vlies- oder vliesstoffförmige Struktur aufweisen oder in Form eines Vlieses, insbesondere Vliesstoffes, ausgebildet sein.

Das Material ist in einer weiteren Ausführungsform frei von nichtkollagenen Bestandteilen, insbesondere frei von nichtkollagenen Proteinen.

Bevorzugt handelt es sich bei dem Material um ein Biopolymer, d.h. um ein natürlich vorkommendes Polymer.

Besonders bevorzugt handelt es sich bei dem Material, insbesondere Biopolymer, um ein Protein, insbesondere extrazelluläres Protein.

Das Protein ist vorzugsweise ausgewählt aus der Gruppe bestehend aus Kollagen, Gelatine, Elastin, Retikulin, Fibrin, Fibronektin, Laminin, Albumin und Mischungen aus wenigstens zwei der genannten Proteine.

In einer besonders bevorzugten Ausführungsform handelt es sich bei dem Material, insbesondere Protein, um Kollagen, insbesondere um Kollagen Typ I, Kollagen Typ III oder um eine Mischung aus Kollagen Typ I und Kollagen Typ III.

In einer weiteren Ausführungsform handelt es sich bei dem Material um eine extrazelluläre Matrix eines biologischen Gewebes. Unter dem Ausdruck "biologisches Gewebe" soll im Sinne der vorliegenden Erfindung insbesondere ein tierisches Gewebe, d.h. ein in Tieren vorkommendes Gewebe, oder ein mittels Geweberekonstruktionstechniken (tissue engineering) hergestelltes Gewebe verstanden werden.

Bevorzugt handelt es sich bei dem Material um eine extrazelluläre Matrix eines tierischen oder xenogenen, insbesondere bovinen, equinen oder porcinen, Gewebes.

In einer weiteren Ausführungsform handelt es sich bei dem Material um ein biologisches, insbesondere tierisches oder xenogenes, bevorzugt bovines, equines oder porcines, Gewebe. Besonders bevorzugt ist ein bovines Gewebe.

In einer weiteren Ausführungsform ist das Material aus einem biologischen, insbesondere tierischen oder xenogenen, bevorzugt bovinen, equinen oder porcinen, Gewebe hergestellt. Besonders bevorzugt ist das Material aus einem bovinen Gewebe hergestellt.

Bevorzugt handelt es sich bei dem Material, insbesondere biologischen Gewebe, um Perikard, Pericardium fibrosum, Pericardium serosum, Epikard oder eine Mischung aus wenigstens zwei der genannten Materialien, insbesondere Gewebe.

Gemäß einer besonders bevorzugten Ausführungsform handelt es sich bei dem Material, insbesondere biologischen Gewebe, um Perikard, bevorzugt um bovines Perikard, d.h. Rinderperikard.

Ein derartiges Material wird von der Anmelderin bereits unter der Bezeichnung Lyoplant^{®} kommerziell für den Duraersatz angeboten. Hierbei handelt es sich um ein reines, aus Rinderperikard hergestelltes Kollagen-Implantat.

Die Erfindung beruht insbesondere auf der überraschenden Erkenntnis, dass sich Kollagen, bevozugt Perikard, als Implantatmaterial in besonderer Weise für die Behandlung eines Azetabulumdefekts eignet. Da Kollagen bzw. Perikard ein körperaffiner Stoff darstellt, ist ein hoher Grad an Biokompatibilität gegeben. Weiterhin stellt Kollagen bzw. Perikard ein biologisch abbaubares Material bzw. Gewebe dar, wodurch die Neogenese von Knochengewebe gefördert und mithin die Knochendefektgröße minimiert werden kann ("defect downsizing"). Die Neogenese des Knochengewebes wird dabei insbesondere durch die Faserstruktur von Kollagen bzw. Perikard begünstigt. Des Weiteren stellt Kollagen bzw. Perikard ein bearbeitbares und insbesondere formbares Material bzw. Gewebe dar, welches problemlos an patientienindividuelle Knochendefektgrößen und -geometrien angepasst werden kann. Zusätzlich stellt Kollagen bzw. Perikard ein zugfestes Material bzw. Gewebe dar, so dass es auch lasttragende Funktionen erfüllen kann. Sollte eine Revision erforderlich sein, kann eventuell noch im Defektbereich vorhandenes Kollagen bzw. Perikard im Körper des Patienten verbleiben, ohne dass sich dies nachteilig auf den Revisionserfolg auswirkt.

In einer weiteren Ausführungsform handelt es sich bei dem Material, insbesondere Biopolymer, um ein Polysaccharid, insbesondere Mucopolysaccharid, vorzugsweise ausgewählt aus der Gruppe bestehend aus Stärke, Amylose, Amylopektin, Dextran, Dextrin, Cellulose, Methylcellulose, Ethylcellulose, Hydroxymethylcellulose, Hydroxyethylcellulose, Hydroxypropylcellulose, Carboxymethylcellulose, Carboxyethylcellulose, Chitin, Chitosan, Hyaluronsäure, Dextransulfat, Heparin, Heparansulfat, Chondroitinsulfat, Dermatansulfat, Keratansulfat und Mischungen aus wenigstens zwei der genannten Polysaccharide.

In einer weiteren Ausführungsform handelt es sich bei dem Material um ein synthetisches Polymer, d.h. um ein künstlich, beispielsweise in einem chemischen Labor oder in einer vergleichbaren Einrichtung, hergestelltes Polymer.

Unter dem Ausdruck "Polymer" soll im Sinne der vorliegenden Erfindung ein Homo- oder Copolymer verstanden werden.

Unter dem Ausdruck "Copolymer" soll im Sinne der vorliegenden Erfindung ein Polymer verstanden werden, welches aus wenigstens zwei verschiedenen Monomereinheiten zusammengesetzt ist.

Bei dem synthetischen Polymer kann es sich grundsätzlich um ein segmentiertes Polymer bzw. Blockpolymer, ein statistisches bzw. randomisiertes Polymer, ein isotaktisches Polymer, ein syndiotaktisches Polymer oder ein ataktisches Polymer handeln.

In einer weiteren Ausführungsform handelt es sich bei dem synthetischen Polymer um ein Polyhydroxyalkanoat.

Insbesondere kann das synthetische Polymer ausgewählt sein aus der Gruppe bestehend aus Polyglycolid, Polylactid, Poly-ε-caprolacton, Polytrimethylencarbonat, Poly-p-dioxanon (Poly-1,4-dioxan-2-on), Poly-3-hydroxybutyrat, Poly-4-hydroxybutyrat, Copolymere aus wenigstens zwei der genannten Polymere und Blends aus wenigstens zwei der genannten Polymere.

In einer weiteren Ausführungsform liegt das wenigstens eine Flächengebilde, insbesondere das Material, in lyophilisierter, d.h. gefriergetrockneter, Form vor.

In einer weiteren Ausführungsform handelt es sich bei dem Material um ein Metall, insbesondere Tantal.

In einer weiteren Ausführungsform weist das wenigstens eine Flächengebilde eine beliebige Kombination oder Mischung der in den vorherigen Ausführungsformen beschriebenen Materialien auf.

In einer weiteren Ausführungsform weist das Material einen Anteil von 10 Gew.-% bis 95 Gew.-%, insbesondere 15 Gew.-% bis 90 Gew.-%, vorzugsweise 20 Gew.-% bis 80 Gew.-%, auf, bezogen auf das Gesamtgewicht des wenigstens einen Flächengebildes.

In einer weiteren Ausführungsform besteht das wenigstens eine Flächengebilde aus dem in vivo abbaubaren (degradierbaren) oder in vivo resorbierbaren Material.

Grundsätzlich kann das wenigstens eine Flächengebilde wenigstens teilweise, insbesondere vollständig, textil ausgestaltet sein. Zum Beispiel kann das wenigstens eine Flächengebilde wenigstens teilweise, insbesondere vollständig, in Form eines Gewebes, Gewirks wie Nähgewirks, Gestricks, Geflechts, Vlieses, Vliesstoffs, Netzes wie gewirkten Netzes, Filzes oder Maschenstoffes vorliegen.

Bevorzugt handelt es sich bei dem wenigstens einen Flächengebilde um ein textiles Flächengebilde, insbesondere um ein gewebtes, gewirktes, gestricktes, geflochtenes, vliesförmiges, vliesstoffförmiges, netzförmiges, filzförmiges oder maschenstoffförmiges Flächengebilde.

Erfindungsgemäß ist es jedoch besonders bevorzugt, wenn das wenigstens eine Flächengebilde wenigstens teilweise, insbesondere vollständig, nicht-textil ausgestaltet ist. Bevorzugt weist das wenigstens eine Flächengebilde eine nicht-textile Faserstruktur, insbesondere eine nicht-textile und randomisierte Faserstruktur, auf.

Das wenigstens eine Flächengebilde ist in einer zweckmäßigen Ausführungsform flexibel, insbesondere biegsam, ausgebildet. Auf diese Weise ist eine Anpassung des wenigstens einen Flächengebildes und somit des Implantats an patientenindividuelle Azetabula ohne größere Schwierigkeiten möglich.

In einer weiteren Ausführungsform weist das wenigstens eine Flächengebilde eine variierende, d.h. nichtkonstante, Schichtdicke auf.

Das wenigstens eine Flächengebilde kann insbesondere eine Dicke von 0,1 mm bis 5 mm, insbesondere 0,2 mm bis 3,5 mm, vorzugsweise 0,5 mm bis 3,0 mm, aufweisen.

In einer weiteren Ausführungsform weist das wenigstens eine Flächengebilde ein Flächengewicht von 0,1 mg/cm² bis 8000 mg/cm², insbesondere 0,5 mg/cm² bis 6000 mg/cm², vorzugsweise 0,5 mg/cm² bis 5000 mg/cm², auf.

In einer weiteren Ausführungsform weist das Material eine in vivo Abbau- bzw. Resorptionszeit von 1 Monat bis 15 Jahre, insbesondere 2 Monate bis 10 Jahre, vorzugsweise 6 Monate bis 5 Jahre, auf.

Bevorzugt weist das wenigstens eine Flächengebilde eine Zugfestigkeit von 10 N/mm² bis 800 N/mm², insbesondere 15 N/mm² bis 500 N/mm², vorzugsweise 30 N/mm² bis 700 N/mm², auf.

Unter dem Ausdruck "Zugfestigkeit" soll im Sinne der vorliegenden Erfindung die maximale mechanische Zugspannung verstanden werden, welche das wenigstens eine Flächengebilde aushält, bevor es bricht bzw. reißt. Die Zugfestigkeit kann im Zugversuch aus der maximal erreichten Zugkraft, bezogen auf den ursprünglichen Querschnitt des wenigstens einen Flächengebildes errechnet werden. Im Falle eines metallischen Materials lässt sich die Zugfestigkeit des wenigstens einen Flächengebildes beispielsweise mittels eines gemäß EN ISO 6892-1 oder ISO 6892 genormten Zugversuchs ermitteln. Im Falle eines polymeren Materials, insbesondere Kunststoffmaterials, lässt sich die Zugfestigkeit des wenigstens einen Flächengebildes beispielsweise mittels eines gemäß ISO 527 oder ASTM D 638 genormten Zugversuchs ermitteln. In dem Fall, dass das Material ein biologisches Gewebe, insbesondere Perikard, ist, lässt sich die Zugfestigkeit des wenigstens einen Flächengebildes beispielsweise mittels eines gemäß ISO 9073-3 genormten Zugversuchs ermitteln.

In einer weiteren Ausführungsform weist das wenigstens eine Flächengebilde wenigstens ein Loch, wenigstens eine Perforation oder wenigstens einen Schlitz auf. Auf diese Weise kann das Einwachsen von Knochenneugewebe durch das wenigstens eine Flächengebilde hindurch und mithin ein Einwachsen von Knochenneugewebe in die Defektzone begünstigt werden. Ferner ist hierdurch beispielsweise eine in vivo Befestigung des Implantats, insbesondere mittels Knochenschrauben oder Knochennägel, erzielbar.

Der Ausdruck "wenigstens ein Loch", "wenigstens eine Perforation" bzw. "wenigstens ein Schlitz" kann im Sinne der vorliegenden Erfindung ein Loch, eine Perforation bzw. ein Schlitz oder mehrere, d.h. zwei oder mehr Löcher, Perforationen oder Schlitze bedeuten.

Bevorzugt handelt es sich bei dem wenigstens einen Loch, der wenigstens einen Perforation oder dem wenigstens einen Schlitz um wenigstens ein die Dicke des wenigstens einen Flächengebildes durchbrechendes Loch, um wenigstens eine die Dicke des wenigstens einen Flächengebildes durchbrechende Perforation bzw. um wenigstens einen die Dicke des wenigstens einen Flächengebildes durchbrechenden Schlitz.

Das wenigstens eine Loch kann insbesondere in Form wenigstens einer Öse gestaltet sein. Besonders bevorzugt weist das wenigstens eine Flächengebilde eine Vielzahl von Löchern, Perforationen oder Schlitzen, insbesondere mehrere, die Dicke des wenigstens einen Flächengebildes durchbrechende Löcher, Perforationen oder Schlitze, auf.

In einer weiteren Ausführungsform ist das wenigstens eine Flächengebilde offenporig ausgestaltet. Das wenigstens eine Flächengebilde kann insbesondere einen mittleren Porendurchmesser von 10 µm bis 5000 µm, insbesondere 15 µm bis 4000 µm, vorzugsweise 100 µm bis 3000 µm, aufweisen. Durch eine offenporige Ausgestaltung des wenigstens einen Flächengebildes kann die Bildung von Knochenneugewebe zusätzlich stimuliert werden.

Das wenigstens eine Flächengebilde weist einen zentralen Bereich auf.

Das wenigstens eine Flächengebilde weist drei längliche Funktionsbereiche, auf.

Unter dem Ausdruck "länglicher Funktionsbereich" bzw. "längliche Funktionsbereiche" soll bzw. sollen im Sinne der vorliegenden Erfindung ein Teilbereich bzw. Teilbereiche des wenigstens einen Flächengebildes verstanden werden, welcher bzw. welche eine im Verhältnis zu einem anderen Teilbereich, insbesondere einem zentralen Bereich, des wenigstens einen Flächengebildes größere Länge besitzt bzw. besitzen.

Der Funktionsbereich ist vorzugsweise dazu eingerichtet, eine Befestigung des wenigstens einen Flächengebildes zu bewirken, insbesondere an Knochengewebe, welches an das Azetabulum oder an einen Azetabulumdefekt angrenzt. Der Funktionsbereich kann im Sinne der vorliegenden Erfindung daher auch als" Befestigungsbereich" bezeichnet werden. Entsprechend kann der Ausdruck" Funktionsbereich" im Sinne der vorliegenden Erfindung mehrere Befestigungsbereiche bedeuten.

Das wenigstens eine Flächengebilde weist drei längliche, bevorzugt quader- oder streifenförmige, Funktionsbereiche auf.

Die Funktionsbereiche erstrecken sich aus dem zentralen Bereich des wenigstens einen Flächengebildes.

Insbesondere können die Funktionsbereiche radial um den zentralen Bereich des wenigstens einen Flächengebildes angeordnet sein.

Wie bereits erwähnt, sind die Funktionsbereiche bevorzugt streifenförmig gestaltet, wobei freie Enden der Funktionsbereiche eckig oder eckenlos ausgebildet sein können.

Der zentrale Bereich ist vorzugsweise in Form eines kreis-, oval- oder ellipsenzylindrischen Körpers, insbesondere in Form einer Scheibe, ausgebildet.

In einer weiteren Ausführungsform ist der zentrale Bereich, insbesondere nur der zentrale Bereich, mehrlagig, insbesondere doppellagig, ausgebildet.

In einer weiteren Ausführungsform sind die Funktionsbereiche, insbesondere nur die Funktionsbereiche, mehrlagig, insbesondere doppellagig, ausgebildet.

Das wenigstens eine Flächengebilde ist in einer weiteren Ausführungsform nach Art eines dreiflügeligen Propellers oder dreiblättrigen Kleeblatts gestaltet.

In einer weiteren Ausführungsform ist das wenigstens eine Flächengebilde einteilig aufgebaut, d.h. aus einem Stück des Materials gefertigt.

In einer alternativen Ausführungsform ist das wenigstens eine Flächengebilde mehrteilig aufgebaut, d.h. das wenigstens eine Flächengebilde ist aus mehreren Stücken des Materials oder aus mehreren Stücken, welche sich hinsichtlich des Materials voneinander unterscheiden, gefertigt, wobei die Teile des wenigstens einen Flächengebildes, insbesondere der oben erwähnte zentrale Bereich sowie die oben erwähnten Funktionsbereiche, miteinander verbunden, insbesondere miteinander vernäht oder stoffschlüssig miteinander verbunden, beispielsweise miteinander verklebt, sein können. Dabei können die Teile, insbesondere der zentrale Bereich und die Funktionsbereiche, gleich oder verschieden ausgebildet sein. Erfindungsgemäß kann es beispielsweise denkbar sein, dass der zentrale Bereich und die Funktionsbereiche unterschiedlich ausgebildet sind, wobei die Funktionsbereiche untereinander gleich ausgebildet sind. Bezüglich möglicher Ausgestaltungen von Teilen eines mehrteilig aufgebauten Flächengebildes bzw. mehrteilig aufgebauter Flächengebilde wird vollständig auf die im Zusammenhang mit dem wenigstens einen Flächengebilde sowie auf die noch folgenden Ausführungen zu einem optional vorhandenen textilen Netz Bezug genommen.

In einer weiteren Ausführungsform weist das Implantat mehrere, bevorzugt zwei, Flächengebilde auf, welche jeweils wenigstens ein teilweise in vivo abbaubares (degradierbares) oder ein wenigstens teilweise in vivo resorbierbares Material, insbesondere ein nur teilweise in vivo abbaubares oder nur teilweise in vivo resorbierbares Material oder ein vollständig in vivo abbaubares oder vollständig in vivo resorbierbares Material, aufweisen. Dabei können sich die Flächengebilde in Bezug auf das Material voneinander unterscheiden. Erfindungsgemäß ist es jedoch bevorzugt, wenn die Flächengebilde das gleiche Material aufweisen. Bezüglich des Materials wird vollständig auf die bisherige Beschreibung Bezug genommen. Die dort in Bezug auf das wenigstens eine Flächengebilde beschriebenen Materialien gelten auch für den Fall, dass das Implantat mehrere Flächengebilde aufweist.

Weiterhin können die Flächengebilde bezüglich ihrer Form oder Struktur gleich oder verschieden ausgebildet sein. Beispielsweise können die Flächengebilde jeweils textil oder jeweils nicht-textil ausgestaltet sein. Alternativ kann das Implantat wenigstens ein textiles Flächengebilde und wenigstens ein nichttextiles Flächengebilde aufweisen. Insoweit wird ebenfalls vollständig auf die bisherige Beschreibung Bezug genommen. Die dort insbesondere im Zusammenhang mit dem wenigstens einen Flächengebilde beschriebenen textilen und nichttextilen Formen bzw. Strukturen gelten auch für den Fall, dass das Implantat mehrere Flächengebilde aufweist.

Zum Beispiel kann das Implantat ein Flächengebilde aus Rinderperikard, insbesondere lyophilisiertem Rinderperikard, und ein netzförmiges, insbesondere gewirktes, Flächengebilde, beispielsweise aus Polyglycolidfäden oder Fäden aus einem Terpolymer, welches aus Glycolid-, ε-Caprolacton- und Trimethylencarbonateinheiten zusammengesetzt ist, aufweisen.

Die Flächengebilde sind in einer weiteren Ausführungsform übereinander angeordnet.

Besonders bevorzugt sind die Flächengebilde, vorzugsweise übereinander angeordnete Flächengebilde, miteinander verbunden, insbesondere randseitig, bevorzugt nur randseitig, miteinander verbunden. Dabei können die Flächengebilde beispielsweise miteinander vernäht oder stoffschlüssig miteinander verbunden sein, insbesondere miteinander verklebt sein. Die Flächengebilde können beispielsweise mittels eines Gewebeklebers, wie beispielsweise mittels Fibrin-Kleber, miteinander verbunden sein.

Besonders bevorzugt schließen unmittelbar benachbarte Flächengebilde, insbesondere unmittelbar übereinander angeordnete Flächengebilde, einen Hohlraum (Kavität) ein.

In einer bevorzugten Ausführungsform weist jedes Flächengebilde einen zentralen Bereich und wenigstens einen länglichen, insbesondere streifenförmigen, Funktionsbereich, insbesondere Befestigungsbereich, auf.

Gemäß einer besonders bevorzugten Ausführungsform weist jedes Flächengebilde einen zentralen Bereich und mehrere längliche, insbesondere streifenförmige, Funktionsbereiche, insbesondere Befestigungsbereiche, auf. Insbesondere können die Funktionsbereiche, insbesondere Befestigungsbereiche, radial um den zentralen Bereich angeordnet sein.

In einer weiteren Ausführungsform sind die Flächengebilde derart übereinander angeordnet, dass längliche Funktionsbereiche eines Flächengebildes versetzt zu länglichen Funktionsbereichen eines darunter oder darüber angeordneten Flächengebildes angeordnet sind.

Bezüglich weiterer Merkmale und Vorteile der Flächengebilde wird vollständig auf die im Rahmen des wenigstens einen Flächengebildes gemachten Ausführungen Bezug genommen.

Die dort gemachten Ausführungen gelten auch für den Fall, dass das Implantat mehrere Flächengebilde aufweist.

Das Implantat, insbesondere das wenigstens eine Flächengebilde, weist in einer weiteren Ausführungsform ferner einen Zusatzstoff auf.

Der Zusatzstoff kann sich insbesondere zwischen zwei benachbarten, insbesondere zwischen zwei übereinander angeordneten und vorzugsweise miteinander verbundenen, insbesondere randseitig miteinander verbundenen, Flächengebilden befinden.

Bevorzugt befindet sich der Zusatzstoff zwischen zentralen Bereichen, insbesondere nur zwischen zentralen Bereichen von übereinander angeordneten und vorzugsweise miteinander verbundenen, insbesondere randseitig miteinander verbundenen, Flächengebilden.

Alternativ bevorzugt befindet sich der Zusatzstoff zwischen länglichen Funktionsbereichen, insbesondere nur zwischen länglichen Funktionsbereichen, von übereinander angeordneten und vorzugsweise miteinander verbundenen, insbesondere randseitig miteinander verbundenen, Flächengebilden.

Der Zusatzstoff kann einen Anteil von 2 Gew.-% bis 95 Gew.-%, insbesondere 5 Gew.-%bis 90 Gew.-%, bevorzugt 5 Gew.-% bis 50 Gew.-%, aufweisen, bezogen auf das Gesamtgewicht des Implantats.

Der Zusatzstoff liegt in einer weiteren Ausführungsform in Form von Partikeln, insbesondere in Form eines Granulats, vor. Die Partikeln weisen vorzugsweise wenigstens eine Abmessung in einem Größenbereich von 50 µm bis 5 mm, insbesondere 0,5 mm bis 4 mm, bevorzugt 1 mm bis 2 mm, auf. Bei der Abmessung kann es sich insbesondere um die Länge, die Höhe, die Dicke und/oder den Durchmesser, insbesondere mittleren Durchmesser, der Partikel handeln.

In einer weiteren Ausführungsform ist der Zusatzstoff ausgewählt aus der Gruppe bestehend aus antimikrobieller, insbesondere antibiotischer, Zusatzstoff, osteokonduktiver Zusatzstoff, osteoinduktiver Zusatzstoff und Mischungen aus wenigstens zwei der genannten Zusatzstoffe.

Der antimikrobielle, insbesondere antibiotische, Zusatzstoff kann insbesondere ausgewählt sein aus der Gruppe bestehend aus Silber, Kupfer, Zink, Chlorhexidin Triclosan, Polyhexanid (PHMB) und Mischungen aus wenigsten zwei der genannten antimikrobiellen, insbesondere antibiotischen, Zusatzstoffe.

Der osteokonduktive Zusatzstoff kann insbesondere ausgewählt sein aus der Gruppe bestehend aus Calciumsulfat, Hydroxyapatit, Calciumphosphat, α-Tricalciumphosphat, Kollagen, Kollagen-Hydroxyapatit-Mischung, Silikon-Hydroxyapatit, Magnesium-Hydroxyapatit, bioaktives Glas, Knochenspäne oder -chips und Mischungen aus wenigstens zwei der genannten osteokonduktiven Zusatzstoffe.

Der osteoinduktive Zusatzstoff kann insbesondere ausgewählt sein aus der Gruppe bestehend aus BMP1 (knochenmorphogenetisches Protein 1), BMP2 (knochenmorphogenetisches Protein 2), BMP3 (knochenmorphogenetisches Protein 3), BMP4 (knochenmorphogenetisches Protein 4), BMP5 (knochenmorphogenetisches Protein 5), BMP6 (knochenmorphogenetisches Protein 6), BMP7 (knochenmorphogenetisches Protein 7), BMP8A (knochenmorphogenetisches Protein 8A), BMP8B (knochenmorphogenetisches Protein 8B), BMP10 (knochenmorphogenetisches Protein 10), BMP15 (knochenmorphogenetisches Protein 15), GDF10 (growth differentiation factor 10) und Mischungen aus wenigstens zwei der genannten osteoinduktiven Zusatzstoffe.

In einer weiteren Ausführungsform weist das Implantat ferner ein textiles, insbesondere gewirktes, Netz auf. Das Netz ist vorzugsweise dazu eingerichtet, eine Armierung des Implantats zu bewirken.

Das Netz kann insbesondere ein in vivo abbaubares oder in vivo resorbierbares Material aufweisen oder aus einem solchen Material bestehen. Das Material kann beispielsweise aus der Gruppe bestehend aus Polyhydroxyalkanoat, Polyglycolid, Polylactid, Poly-ε-caprolacton, Polytrimethylencarbonat, Poly-p-dioxanon (Poly-1,4-dioxan-2-on), Poly-3-hydroxybutyrat, Poly-4-hydroxybutyrat, Copolymere aus wenigstens zwei der genannten Polymere und Blends aus wenigstens zwei der genannten Polymere ausgewählt sein. Insbesondere kann es sich bei dem in vivo abbaubaren oder resorbierbaren Material um ein Terpolymer, insbesondere Terblockpolymer, handeln, welches aus Polyglykolideinheiten, Poly-ε-caprolactoneinheiten und Polytrimethylencarbonateinheiten zusammengesetzt ist.

Alternativ kann das Netz ein in vivo nicht abbaubares oder in vivo nicht resorbierbares Material aufweisen oder aus einem solchen Material bestehen. Bei dem Material kann es sich insbesondere um ein Polyolefin, wie beispielsweise Polyethylen, insbesondere ultrahochmolekulares Polyethylen (UHMWPE), oder Polypropylen, oder um einen Polyester, wie beispielsweise Polyethylenterephthalat (PET), handeln.

Insbesondere kann es sich bei dem Netz um das von der Anmelderin unter der Bezeichnung "Optilene^{®} Mesh" oder "Optilene^{®} Mesh Elastic" kommerziell vertriebene Netz handeln. Bei dem Netz "Optilene^{®}Mesh" handelt es sich um ein Netz aus monofilen Polypropylenfäden, wobei das Netz ein Flächengewicht von ca. 60 g/m² und eine Porengröße von ca. 1.5 mm aufweist. Bei dem Netz "Optilene^{®} Mesh Elastic" handelt es sich um ein Netz aus monofilen Polypropylenfäden, wobei das Netz ein Flächengewicht von ca. 48 g/m² und eine Porengröße von 3.6 x 2.8 mm aufweist.

Bei dem Implantat handelt es sich um ein Implantat zur Verwendung bei der Behandlung und/oder Rekonstruktion, insbesondere Auskleidung und/oder Abdichtung und/oder Unterfütterung und/oder wenigstens teilweisen Auffüllung, eines Azetabulumdefekts.

In einer weiteren Ausführungsform handelt es sich bei dem Implantat um ein Implantat zur Verwendung als Gegenlager für ein Knochenfüllmaterial bei der Behandlung eines Azetabulumdefekts. Bei dem Knochenfüllmaterial kann es sich dabei um ein natürliches Knochenfüllmaterial, beispielsweise um Knochenspäne oder -chips, oder um ein synthetisches, d.h. künstliches, Knochenfüllmaterial, beispielsweise um Calciumsulfat, Hydroxyapatit, Calciumphosphat, α-Tricalciumphosphat, Kollagen, Kollagen-Hydroxyapatit-Mischung, Silikon-Hydroxyapatit, Magnesium-Hydroxyapatit, bioaktives Glas oder um eine Mischung aus wenigstens zwei der genannten künstlichen Knochenfüllmaterialien, handeln.

In einer weiteren Ausführungsform handelt es sich bei der Behandlung um eine Revision.

Die Erfindung betrifft gemäß einem zweiten Aspekt ein chirurgisches Kit, vorzugsweise zum Behandeln und/oder Rekonstruieren, insbesondere Auskleiden und/oder Abdichten und/oder Unterfüttern und/oder wenigstens teilweisen Auffüllen, eines Azetabulumdefekts.

Das Kit weist ein Implantat gemäß erstem Erfindungsaspekt auf.

Zusätzlich weist das Kit wenigstens eine Komponente auf, welche ausgewählt ist aus der Gruppe bestehend aus Knochenfüllmaterial, textiles Netz, Knochenkleber, Endoprothese, Abstützschale, Spacer-Implantat und Befestigungselement.

Bei dem Knochenfüllmaterial kann es sich grundsätzlich um ein natürliches Knochenfüllmaterial, beispielsweise um Knochenspäne oder -chips, oder um ein synthetisches Knochenfüllmaterial, beispielsweise um Calciumsulfat, Hydroxyapatit, Calciumphosphat, α-Tricalciumphosphat, Kollagen, Kollagen-Hydroxyapatit-Mischung, Silikon-Hydroxyapatit, Magnesium-Hydroxyapatit, bioaktives Glas oder eine Mischung aus wenigsten zwei der genannten Knochenfüllmaterialien, handeln.

Bei dem textilen Netz kann es sich beispielsweise um ein gewirktes Netz, insbesondere aus Polypropylen, handeln. Bezüglich weiterer Merkmale und Vorteile des textilen Netzes wird vollständig auf die im Rahmen des ersten Erfindungsaspektes gemachten Ausführungen Bezug genommen. Die dort in Bezug auf das textile Netz gemachten Ausführungen gelten (sinngemäß) auch für das chirurgische Kit.

Bei dem Knochenkleber kann es sich beispielsweise um einen aushärtbaren Knochenzement handeln.

Bei der Endoprothese handelt es sich vorzugsweise um eine künstliche Gelenkpfanne oder ein künstliches Gelenkpfannen-Inlay.

Bei dem Befestigungselement kann es sich beispielsweise um eine Knochenschraube oder einen Mark- bzw. Knochennagel handeln. Weiterhin kann es sich bei dem Befestigungselement um ein Nahtmaterial, vorzugsweise um ein nicht resorbierbares Nahtmaterial, beispielsweise aus Polypropylen oder ultrahochmolekulares Polyethylen (UHMWPE), handeln.

Bezüglich weiterer Merkmale und Vorteile des chirurgischen Kits, insbesondere des Implantats, wird zur Vermeidung von Wiederholungen vollständig auf die im Rahmen des ersten Erfindungsaspekts gemachten Ausführungen Bezug genommen, welche (sinngemäß) auch für das chirurgische Kit gelten.

Weitere Vorteile und Merkmale der Erfindung ergeben sich aus den Ansprüchen sowie aus der nachfolgenden Beschreibung bevorzugter Ausführungsformen anhand von Figurenbeschreibungen und der dazugehörigen Figuren. Dabei können Merkmale der Erfindung jeweils für sich alleine oder in Kombination miteinander verwirklicht sein. Die nachfolgend beschriebenen Ausführungsformen dienen der weiteren Erläuterung der Erfindung, ohne diese hierauf zu beschränken.

### KURZBESCHREIBUNG DER FIGUREN

In den Figuren ist schematisch Folgendes gezeigt:
- Fig. 1:: eine Draufsicht auf ein menschliches Azetabulum,
- Fig. 2a:: eine Ausführungsform eines nicht erfindungsgemäßen Implantats,
- Fig. 2b:: eine Ausführungsform eines erfindungsgemäßen Implantats,
- Fig. 2c:: eine Ausführungsform eines nicht erfindungsgemäßen Implantats,
- Fig. 2d:: eine Ausführungsform eines nicht erfindungsgemäßen Implantats,
- Fig. 2e:: eine Ausführungsform eines erfindungsgemäßen Implantats,
- Fig. 2f:: eine Ausführungsform eines nicht erfindungsgemäßen Implantats und
- Fig. 2g:: eine Ausführungsform eines erfindungsgemäßen Implantats.

### AUSFÜHRLICHE FIGURENBESCHREIBUNG

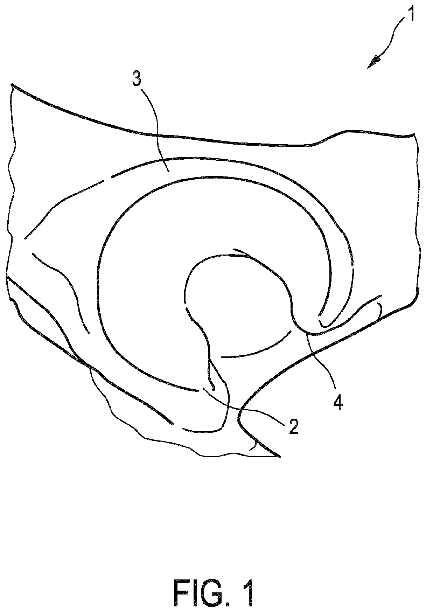

Fig. 1 zeigt schematisch eine Draufsicht auf ein menschliches Azetabulum 1, auch als Hüftgelenk- oder Beckenpfanne bezeichnet. Hierbei handelt es sich um den vom Becken gebildeten knöchernen Anteil des Hüftgelenks. Das Azetabulum entsteht durch Verschmelzung von Teilen des Sitz-, Darm- und Schambeins. Die Verschmelzung ist mit ca. 6 Monaten abgeschlossen.

Bei idealen Voraussetzungen besteht eine Übereinstimmung zwischen Azetabulum und Femurkopf, d.h. der runde Femurkopf passt genau in das Azetabulum, welches ihn weitgreifend einbettet und umschließt. Das Hüftgelenk ist als vielachsiges Kugelgelenk ausgebildet und dadurch in nahezu jede Richtung mehr oder weniger frei beweglich. Dies gewährleistet eine hohe Beweglichkeit und Belastbarkeit.

Die gelenkbildenden Anteile des Hüftgelenks sind von einer bindegewebigen Kapsel umgeben, deren Innenschicht, die Synovia, ständig neue Gelenkflüssigkeit produziert. Eine ringförmige Gelenklippe aus Knorpel bildet den Rand der knöchernen Pfanne.

Das Azetabulum 1 besitzt einen vorderen Pfannenrand 2, auch als sogenanntes Vorderhorn bezeichnet, sowie einen hinteren Pfannenrand 4, auch als sogenanntes Hinterhorn bezeichnet. Das dazwischenliegende Pfannendach 3 ist rund oder im Wesentlichen rund auslaufend ausgebildet.

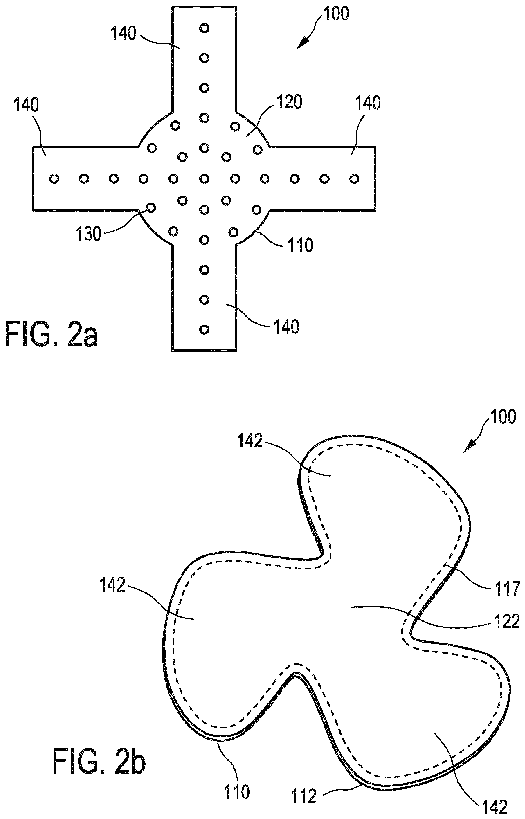

Fig. 2a zeigt schematisch eine Ausführungsform eines nicht erfindungsgemäßen Implantats.

Das in Fig. 2a gezeigte Implantat 100 weist ein Flächengebilde 110 auf. Das Flächengebilde 110 weist einen zentralen Bereich 120 sowie vier längliche Funktionsbereiche 140 auf, welche sich aus dem zentralen Bereich 120 erstrecken.

Der zentrale Bereich 120 ist scheibenförmig gestaltet.

Die Funktionsbereiche 140 sind vorzugsweise streifenförmig gestaltet. Sie sind weiterhin vorzugsweise dazu ausgebildet, eine Befestigung des Implantats 100 an Knochengewebe, welches an ein Azetabulum oder einen Azetabulumdefekt angrenzt, zu bewirken. Die Funktionsbereiche 140 können im Sinne der vorliegenden Erfindung daher auch als Befestigungsbereiche bezeichnet werden.

Um das Knochenwachstum zu erleichtern, kann das Implantat 100 mit Löchern 130 versehen sein, welche die Dicke des Flächengebildes 110 vorzugsweise durchbrechen. Dabei können - wie dargestellt - sowohl der zentrale Bereich 120 als auch die Funktionsbereiche 140 - mit den Löchern 130 versehen sein. Weiterhin können die Löcher 130 zur erleichterten Fixierung des Implantats 100 vorgesehen sein.

Das in Fig. 2b dargestellte Implantat 100 weist zwei Flächengebilde 110; 112 auf, welche randseitig, d.h. entlang der Randzonen der Flächengebilden 110; 112, mittels einer Naht 117 miteinander vernäht sind. Bei der Naht 117 handelt es sich vorzugsweise um ein in vivo abbaubares oder in vivo resorbierbares Nahtmaterial, beispielsweise um einen vorzugsweise geflochtenen Polyglycolidfaden.

Die Flächengebilde 110; 112 weisen jeweils einen zentralen Bereich und drei längliche, sich aus dem zentralen Bereich erstreckende Funktionsbereiche auf. Zu erkennen sind in der Fig. 2b der zentrale Bereich 122 sowie die drei Funktionsbereiche 142 des - vom Betrachters aus gesehen - oberen Flächengebildes 112.

Das Implantat 100 besitzt vorzugsweise die Form eines dreiblättrigen Kleeblatts bzw. eines dreiflügeligen Propellers.

Ein zwischen den Flächengebilden 110; 112 ausgebildeter Hohlraum kann beispielsweise mit einem osteokonduktiven Zusatzstoff und/oder osteoinduktiven Zusatzstoff wenigstens teilweise befüllt sein. Dadurch kann das Einwachsen und die Entstehung von Knochenneugewebe stimuliert werden.

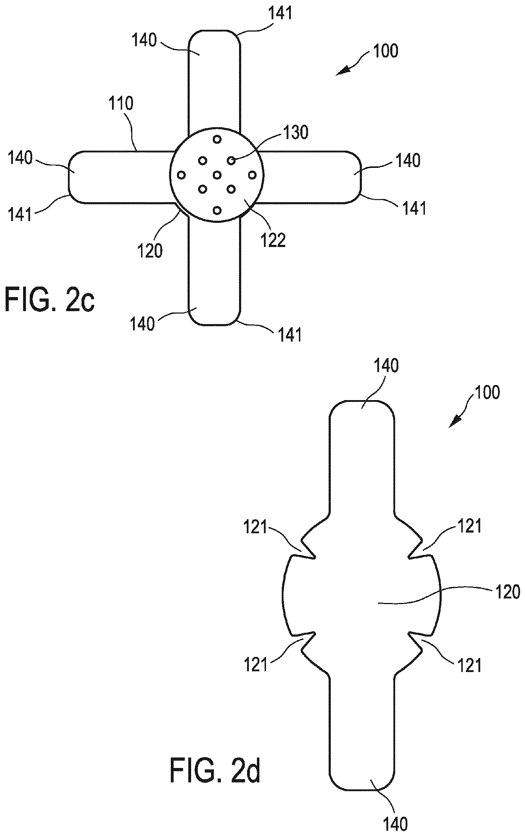

Das in Fig. 2c dargestellte Implantat 100 weist ein Flächengebilde 110 mit einem zentralen und vorzugsweise scheibenförmigen Bereich 120 und vier länglichen, vorzugsweise streifenförmigen, Funktionsbereichen 140 auf. Die Funktionsbereiche 140 erstrecken sich dabei aus dem zentralen Bereich 120. Die freien Enden 141 der Funktionsbereiche 140 sind vorzugsweise eckenlos gestaltet. Der zentrale Bereich 120 ist weiterhin bevorzugt mit einer scheibenförmigen Lage 122 verbunden, insbesondere vernäht oder verklebt. Zur Stimulierung von Knochenwachstum kann sowohl der zentrale Bereich 120 als auch die Lage 122 mit (die Dicke des zentralen Bereichs 120 bzw. der Lage 122 durchbrechenden) Löchern 130 versehen sein. Weiterhin können die Löcher 130 zur erleichterten Fixierung des Implantats 100 vorgesehen sein.

Ein zwischen dem zentralen Bereich 120 und der Lage 122 ausgebildeter Hohlraum kann beispielsweise mit einem osteokonduktiven Zusatzstoff und/oder osteoinduktiven Zusatzstoff wenigstens teilweise befüllt sein. Dadurch kann das Einwachsen und die Entstehung von Knochenneugewebe stimuliert werden.

Das gemäß Fig. 2d dargestellte Implantat 100 weist ein Flächengebilde 110 mit einem zentralen Bereich 120 sowie zwei länglichen Funktionsbereichen 140 auf, welche sich, vorzugsweise auf gegenüberliegenden Seiten des zentralen Bereichs 120, aus dem zentralen Bereich 120 erstrecken. Zur erleichterten Anpassung an ein Azetabulum kann das Implantat 100 entlang seines zentralen Bereichs 120 Kerben 121, insbesondere V-förmige Kerben, aufweisen.

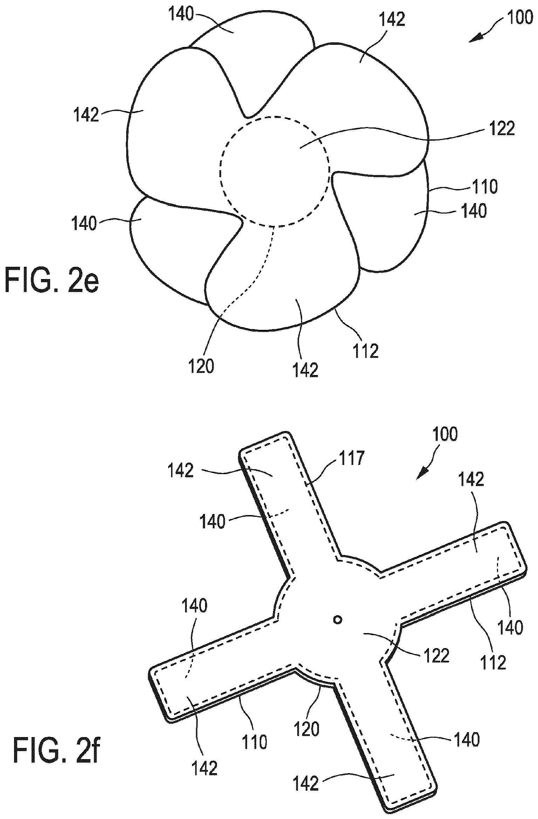

Das Implantat 100 gemäß Fig. 2e weist zwei Flächengebilde 110; 112 auf. Jedes Flächengebilde 110: 112 weist einen zentralen Bereich 120 bzw. 122 sowie drei längliche Funktionsbereiche 140 bzw. 142 auf, welches sie aus dem zentralen Bereich 120 bzw. 122 erstrecken.

Jedes Flächengebilde 110; 112 ist vorzugsweise nach Art eines dreiblättrigen Kleeblatts oder dreiflügeligen Propellerrads ausgestaltet.

Die Flächengebilde 110; 112 sind vorzugsweise derart übereinander angeordnet, dass die Funktionsbereiche 140 versetzt zu den Funktionsbereichen 142 (bzw. umgekehrt) angeordnet sind.

Weiterhin ist es bevorzugt, wenn die beiden zentralen Bereiche 120, 122 randseitig miteinander vernäht sind. Ein hierdurch gebildeter Hohlraum kann beispielsweise mit einem osteokonduktiven Zusatzstoff wenigstens teilweise befüllt sein.

Das gemäß Fig. 2f dargestellte Implantat weist ebenfalls zwei Flächengebilde 110; 112 auf. Die Flächengebilde 110; 112 weisen einen zentralen Bereich 120; 122 und vier längliche, vorzugsweise streifenförmig ausgebildete, Funktionsbereiche 140; 142 auf, welche sich aus dem zentralen Bereich 120; 122 erstrecken. Die beiden Flächengebilden 110; 112 sind mittels einer Naht 117 entlang ihrer Randzonen miteinander vernäht. Ein hierdurch zwischen den Flächengebilden 110; 112 gebildeter Hohlraum kann beispielsweise zur Bewehrung des Implantats 100 ein textiles Netz, insbesondere ein gewirktes Polypropylen-Netz, beispielsweise das von der Anmelderin unter der Bezeichnung "Optilene^{®} Mesh Elastic" vertriebene Netz, enthalten.

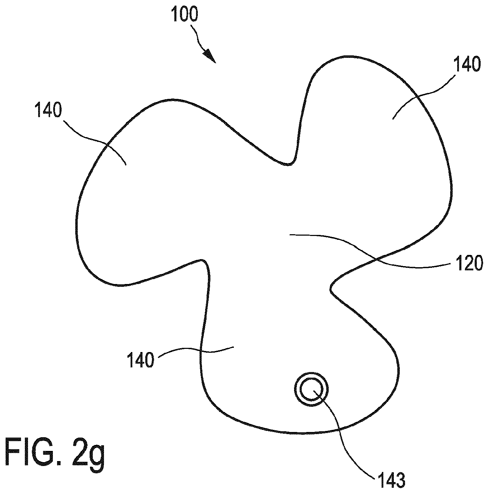

Das gemäß Fig. 2g dargestellte Implantat 100 weist ein Flächengebilde 110 mit einem zentralen Bereich 120 und drei länglichen Funktionsbereichen 140 auf. Die Funktionsbereiche 140 erstrecken sich aus dem zentralen Bereich 120. Insgesamt besitzt das Flächengebilde 110 eine Form, welcher derjenigen eines dreiflügeligen Propellers oder dreiblättrigen Kleeblatts entspricht. Wenigstens einer der Funktionsbereiche 140 kann eine Öse 143 zur Befestigung des Implantats an peripheres Knochengewebe aufweisen.

Die in den Figuren 2a-g dargestellten Implantate weisen ein wenigstens teilweise in vivo abbaubares oder in vivo resorbierbares Material auf. Dabei können insbesondere die in den Figuren dargestellten Flächengebilden entweder vollständig oder nur teilweise ein solches Material aufweisen. Beispielsweise können nur die zentralen Bereiche oder nur die Funktionsbereiche ein wenigsten teilweise in vivo abbaubares oder resorbierbares Material aufweisen oder aus einem solchen Material bestehen. Bei dem Material handelt es sich vorzugsweise um Kollagen, insbesondere um Kollagen Typ I und/oder Kollagen Typ III. Besonders bevorzugt handelt es sich bei dem Material um Perikard, insbesondere Rinderperikard.

Darüber hinaus kommen grundsätzlich jedoch auch andere Materialien in Betracht, welche wenigstens teilweise in vivo abbaubar oder resorbierbar sind. Insoweit wird vollständig auf die in der allgemeinen Beschreibung offenbarten Materialien Bezug genommen.

## Patentansprüche

1. Implantat zur Verwendung bei der Behandlung und/oder Rekonstruktion, insbesondere Auskleidung und/oder Abdichtung und/oder Unterfütterung und/oder wenigstens teilweisen Auffüllung, eines Azetabulumdefekts, aufweisend wenigstens ein Flächengebilde, welches ein wenigstens teilweise in vivo abbaubares oder resorbierbares Material aufweist, **dadurch gekennzeichnet, dass** das wenigstens eine Flächengebilde einen zentralen Bereich und drei längliche Funktionsbereiche aufweist, welche sich aus dem zentralen Bereich erstrecken.

2. Implantat nach Anspruch 1, **dadurch gekennzeichnet, dass** das Material eine randomisierte Faserstruktur, insbesondere Vlies- oder Vliesstoffstruktur, aufweist.

3. Implantat nach Anspruch 1 oder 2, **dadurch gekennzeichnet, dass** das Material frei von nichtkollagenen Bestandteilen, insbesondere frei von nichtkollagenen Proteinen, ist.

4. Implantat nach einem der vorhergehenden Ansprüche, **dadurch gekennzeichnet, dass** es sich bei dem Material um ein Protein, vorzugsweise extrazelluläres Protein, handelt, wobei das Protein vorzugsweise ausgewählt ist aus der Gruppe bestehend aus Kollagen, insbesondere Kollagen Typ I, Kollagen Typ III oder eine Mischung aus Kollagen Typ I und Kollagen Typ III, Gelatine, Elastin, Retikulin, Fibrin, Fibronektin, Laminin, Albumin und Mischungen aus wenigstens zwei der genannten Proteine.

5. Implantat nach einem der vorhergehenden Ansprüche, **dadurch gekennzeichnet, dass** es sich bei dem Material um ein xenogenes, insbesondere bovines, Gewebe, bevorzugt um Perikard, insbesondere Rinderperikard, handelt.

6. Implantat nach einem der Ansprüche 1 bis 3, **dadurch gekennzeichnet, dass** es sich bei dem Material um ein synthetisches Polymer handelt, welches insbesondere ausgewählt ist aus der Gruppe bestehend aus Polyglycolid, Polylactid, Poly-ε-caprolacton, Polytrimethylencarbonat, Poly-3-hydroxybutyrat, Poly-4-hydroxybutyrat, Poly-p-dioxanon, Copolymere davon und Blends aus wenigstens zwei der genannten Polymere, oder dass es sich bei dem Material um ein Metall, insbesondere Tantal, handelt.

7. Implantat nach einem der vorhergehenden Ansprüche, **dadurch gekennzeichnet, dass** das wenigstens eine Flächengebilde in lyophilisierter Form vorliegt.

8. Implantat nach einem der vorhergehenden Ansprüche, **dadurch gekennzeichnet, dass** das wenigstens eine Flächengebilde durchbrechende Löcher, Perforationen oder Schlitze aufweist.

9. Implantat nach einem der vorhergehenden Ansprüche, **dadurch gekennzeichnet, dass** die Funktionsbereiche streifenförmig sind und/oder das Implantat mehrere, vorzugsweise zwei, Flächengebilde aufweist, wobei die Flächengebilde übereinander angeordnet und vorzugsweise randseitig miteinander verbunden, insbesondere vernäht oder verklebt, sind, wobei bevorzugt benachbarte Flächengebilde einen Hohlraum einschließen.

10. Implantat nach einem der vorhergehenden Ansprüche, **dadurch gekennzeichnet, dass** das Implantat ferner einen Zusatzstoff, insbesondere einen osteokonduktiven Zusatzstoff, insbesondere Calciumsulfat, Hydroxyapatit, Calciumphosphat, α-Tricalciumphosphat, Kollagen, Kollagen-Hydroxyapatit-Mischung, Silikon-Hydroxyapatit, Magnesium-Hydroxyapatit, bioaktives Glas, Knochenspäne oder eine Mischung aus wenigstens zwei der genannten Zusatzstoffe, aufweist.

11. Implantat nach Anspruch 10, **dadurch gekennzeichnet, dass** sich der Zusatzstoff zwischen zwei übereinander angeordneten und miteinander verbundenen Flächengebilden befindet.

12. Implantat nach Anspruch 10 oder 11, **dadurch gekennzeichnet, dass** sich der Zusatzstoff zwischen zentralen Bereichen von übereinander angeordneten und miteinander verbundenen Flächengebilden befindet.

13. Implantat nach einem der Ansprüche 10 bis 12, **dadurch gekennzeichnet** sich der Zusatzstoff zwischen Funktionsbereichen von übereinander angeordneten und miteinander verbundenen Flächengebilden befindet.

14. Implantat nach einem der vorhergehenden Ansprüche, **dadurch gekennzeichnet, dass** das Implantat ferner ein textiles, insbesondere gewirktes, Netz aufweist.

15. Chirurgisches Kit, aufweisend ein Implantat nach einem der vorhergehenden Ansprüche und wenigstens eine Komponente ausgewählt aus der Gruppe bestehend aus Knochenfüllmaterial, textiles Netz, Knochenkleber, Endoprothese, Abstützschale, Spacer-Implantat und Befestigungselement.

## Claims

1. Implant for use in treating and/or reconstructing, in particular lining and/or sealing and/or reinforcing and/or at least partially filling, an acetabular defect, comprising at least one flat structure that comprises a material that is at least partially degradable or resorbable in vivo, **characterized in that** the at least one flat structure has a central area and three elongated functional areas that extend out from the central area.

2. Implant according to Claim 1, **characterized in that** the material has a randomized fibre structure, in particular web or nonwoven structure.

3. Implant according to Claim 1 or 2, **characterized in that** the material is free of non-collagenous components, in particular free of non-collagenous proteins.

4. Implant according to any of the preceding claims, **characterized in that** the material is a protein, preferably extracellular protein, wherein the protein is preferably selected from the group consisting of collagen, in particular collagen type I, collagen type III or a mixture of collagen type I and collagen type III, gelatin, elastin, reticulin, fibrin, fibronectin, laminin, albumin and mixtures of at least two of the aforementioned proteins.

5. Implant according to any of the preceding claims, **characterized in that** the material is a xenogeneic, in particular bovine, tissue, preferably pericardium, in particular bovine pericardium.

6. Implant according to any of Claims 1 to 3, **characterized in that** the material is a synthetic polymer selected in particular from the group consisting of polyglycolide, polylactide, poly-ε-caprolactone, polytrimethylene carbonate, poly-3-hydroxybutyrate, poly-4-hydroxybutyrate, poly-p-dioxanone, copolymers thereof and blends of at least two of the aforementioned polymers or **in that** the material is a metal, in particular tantalum.

7. Implant according to any of the preceding claims, **characterized in that** the at least one flat structure is in freeze-dried form.

8. Implant according to any of the preceding claims, **characterized in that** the at least one flat structure has penetrating holes, perforations or slits.

9. Implant according to any of the preceding claims, **characterized in that** the functional areas are strip-shaped and/or the implant comprises a plurality of, preferably two, flat structures, wherein the flat structures are arranged one above the other and are preferably connected to one another at the edges, in particular sewn or adhesively bonded, wherein adjacent flat structures preferably enclose a cavity.

10. Implant according to any of the preceding claims, **characterized in that** the implant further comprises an additive, in particular an osteoconductive additive, in particular calcium sulfate, hydroxyapatite, calcium phosphate, α-tricalcium phosphate, collagen, collagen-hydroxyapatite mixture, silicone hydroxyapatite, magnesium hydroxyapatite, bioactive glass, bone chips or a mixture of at least two of the aforementioned additives.

11. Implant according to Claim 10, **characterized in that** the additive is located between two flat structures that are arranged one above the other and are connected to each other.

12. Implant according to Claim 10 or 11, **characterized in that** the additive is located between central areas of flat structures that are arranged one above the other and are connected to each other.

13. Implant according to any of Claim 10 to 12, **characterized in that** the additive is located between functional areas of flat structures that are arranged one above the other and are connected to each other.

14. Implant according to any of the preceding claims, **characterized in that** the implant further comprises a textile, in particular warp-knitted, mesh.

15. Surgical kit comprising an implant according to any of the preceding claims and at least one component selected from the group consisting of bone filling material, textile mesh, bone adhesive, endoprosthesis, reinforcement cage, spacer implant and fastening element.

## Revendications

1. Implant destiné à l'utilisation dans le traitement et/ou la reconstruction, en particulier le revêtement et/ou l'étanchéification et/ou le rebasage et/ou le comblement au moins partiel, d'un défaut d'acétabulum, comportant au moins une structure plane qui comporte un matériau au moins en partie dégradable ou résorbable in vivo, **caractérisé en ce que** ladite au moins une structure plane présente une zone centrale et trois zones fonctionnelles allongées qui s'étendent à partir de la zone centrale.

2. Implant selon la revendication 1, **caractérisé en ce que** le matériau présente une structure fibreuse randomisée, en particulier une structure de voile ou non-tissé.

3. Implant selon la revendication 1 ou 2, **caractérisé en ce que** le matériau est exempt de composants non collagéniques, en particulier exempt de protéines non collagéniques.

4. Implant selon l'une quelconque des revendications précédentes, **caractérisé en ce que** pour ce qui est du matériau il s'agit d'une protéine, de préférence d'une protéine extracellulaire, la protéine étant choisie de préférence dans le groupe constitué par le collagène, en particulier le collagène de type I, le collagène de type III ou un mélange de collagène de type I et de collagène de type III, la gélatine, l'élastine, la réticuline, le fibrine, la fibronectine, la laminine, l'albumine et des mélanges d'au moins deux des protéines citées.

5. Implant selon l'une quelconque des revendications précédentes, **caractérisé en ce que** pour ce qui est du matériau il s'agit d'un tissu xénogène, en particulier bovin, de préférence d'un péricarde, en particulier d'un péricarde de bœuf.

6. Implant selon l'une quelconque des revendications 1 à 3, **caractérisé en ce que** pour ce qui est du matériau il s'agit d'un polymère synthétique, qui est choisi en particulier dans le groupe constitué par le polyglycolide, le polylactide, la poly-e-caprolactone, le poly(triméthylène carbonate), le poly-3-hydroxybutyrate, le poly-4-hydroxybutyrate, la poly-p-dioxanone, des copolymères de ceux-ci et des alliages d'au moins deux des polymères cités, ou **en ce que** pour ce qui est du matériau il s'agit d'un métal, en particulier du tantale.

7. Implant selon l'une quelconque des revendications précédentes, **caractérisé en ce que** ladite au moins une structure plane se trouve sous forme lyophilisée.

8. Implant selon l'une quelconque des revendications précédentes, **caractérisé en ce que** ladite au moins une structure plane comporte des trous traversants, des perforations ou des fentes.

9. Implant selon l'une quelconque des revendications précédentes, **caractérisé en ce que** les zones fonctionnelles sont en forme de bande et/ou l'implant comporte plusieurs, de préférence deux, structures planes, les structures planes étant superposées, et de préférence liées, en particulier cousues ou collées, entre elles sur les bords, de préférence des structures planes voisines enfermant un espace vide.

10. Implant selon l'une quelconque des revendications précédentes, **caractérisé en ce que** l'implant comporte en outre un additif, en particulier un additif ostéoconducteur, en particulier du sulfate de calcium, de l'hydroxyapatite, du phosphate de calcium, du phosphate tricalcique a, du collagène, un mélange collagène-hydroxyapatite, de l'hydroxyapatite à silicium, de l'hydroxyapatite à magnésium, du verre bioactif, des copeaux osseux ou un mélange d'au moins deux des additifs cités.

11. Implant selon la revendication 10, **caractérisé en ce que** l'additif se trouve entre deux structures planes superposées et liées l'une à l'autre.

12. Implant selon la revendication 10 ou 11, **caractérisé en ce que** l'additif se trouve entre les zones centrales de structures planes superposées et liées entre elles.

13. Implant selon l'une quelconque des revendications 10 à 12, **caractérisé en ce que** l'additif se trouve entre les zones fonctionnelles de structures planes superposées et liées entre elles.

14. Implant selon l'une quelconque des revendications précédentes, **caractérisé en ce que** l'implant comporte en outre un filet textile, en particulier tricoté.

15. Trousse chirurgicale, comportant un implant selon l'une quelconque des revendications précédentes et au moins un composant choisi dans le groupe constitué par un matériau de comblement osseux, un filet textile, un ciment osseux, une endoprothèse, un anneau de soutien, un implant espaceur et un élément de fixation.
